# EUROPEAN PATENT SPECIFICATION

(11) **EP 2 083 917 B1**
(45) Date of publication and mention of the grant of the patent: **21.05.2014**
(21) Application number: 07853064.9
(22) Date of filing: 31.10.2007
(51) Int. Cl.: A61N 5/06

(54) **DISPOSABLE TIP FOR LASER HANDPIECE**
EINWEGSPITZE FÜR EIN LASERHANDSTÜCK
EMBOUT JETABLE POUR PIÈCE À MAIN LASER

(30) Priority: 31.10.2006 US 589799
(43) Date of publication of application: 05.08.2009
(60) Divisional application: 11194015.1
(73) Proprietor: Lumenis Ltd., 20692 Yokneam (IL)
(72) Inventor: RAMSTAD, Paul O., San Jose, CA 95129 (US); ZAKOWSKI, Albert C., San Jose, CA 95132 (US); SMITHSON, Stephen Derek, Redwood City, CA 94061 (US); DEGTYARYOV, Andrey, Sunnyvale, CA 94061 (US); GLUSZCZAK, Michael Robert, San Jose, CA 95123 (US); WHITE, Raymond Gordon, Saratoga, CA 95070 (US); BADER, Ronald S., Boulder Creek, CA 95006 (US); CHILDS, Daniel K., Chicago, IL 60607 (US)
(74) Representative: Virdee-Crofts, Kulwinder Kaur
(86) International application number: PCT/US2007/023091
(87) International publication number: WO 2008/054812

(56) References cited:
- EP-A1- 1 627 662
- WO-A1-01/34048
- US-A1- 2002 081 080
- US-A1- 2002 081 080
- US-A1- 2002 151 887
- US-A1- 2006 095 096
- US-A1- 2006 095 096
- US-A1- 2006 189 964
- US-B1- 6 454 763

## Description

### BACKGROUND OF THE INVENTION

### Field of the Invention

The present invention generally relates to a disposable insert for a laser handpiece. Specifically, the insert provides a hygienic surface for applying monochromatic light to a patient. In particular, the disposable insert is configured for providing vacuum to a laser cavity. More specifically, the insert provides a measure of safety to the user by insuring that the laser will be inoperative in the absence of the insert. By having such an insert, a laser handpiece can apply monochromatic light to a patient with heretofore unrealized results.

### Description of the Related Art

Laser handpieces for applying electromagnetic radiation to a patient's skin for a wide variety of treatments have been long known Patients may have many different types of skin disorders, some of which may be contagious. There is therefore a great need in the skin treatment field for devices which avoid infecting patients when the same device is used on many patients. The US 2006/0189964 A1 discloses a device for insertion into a handpiece according to the preamble of claim 1. Further, in the laser treatment field specifically, there is a great need for safety. In particular, a user of a laser treatment device does not want the laser to fire if there is some component of the device improperly installed. Having a laser misfire could cause injury to the user, the patient, or an assistant standing nearby. There is therefore a great need in the art for maintaining hygienic practices in the skin treatment field as well as providing a measure of safety to a patient. Accordingly, there is now provided with this invention an improved laser radiation treatment system which effectively overcomes the aforementioned difficulties and longstanding problems inherent in skin treatment systems. These problems have been solved in a simple, convenient, and highly effective way by which to have a disposable hygiene shield installed into a laser radiation handpiece.

### SUMMARY OF THE INVENTION

According to one aspect of the invention, a device for insertion into a handpiece comprises a body having a cavity.

The device is configured for insertion into a handpiece having a laser, the device comprising a body, said body comprising: a base; an outer wall extending from a first side of the base and along a periphery of the base to define a cavity within the body; a peripheral flange around a periphery of the outer wall; and at one least channel; said base is of a material transparent to at least one optical wavelength of the laser; said at least one channel is configured to provide vacuum communication between said cavity and said handpiece; wherein the peripheral flange is configured to form a seal around a section of skin tissue when applied to the section of skin tissue; and wherein the body is configured to draw the section of skin tissue into the cavity when a vacuum is pulled through the at least one channel (14a,b) by said handpiece.

The device is characterized in that, the device further comprises a safety interlock for allowing the laser to fire, said safety interlock comprising: at least one pair of pins; wherein said at least pair of pins that, when said device is inserted into said handpiece, said pair of pins provide said safety lock, through one or more of: at least one of said pair of pins is for completing a normally open circuit for allowing the determination of said inserted device; at least one of said pair of pins is for opening a normally closed circuit for allowing the determination of said inserted device; one of said pair of pins is for completing a normally open circuit and the other of said pair of pins opens a normally closed circuit for allowing the determination of the said inserted device; said one pair of pins are for completing a normally open circuit for allowing the determination of the inserted device; said pair of pins are for opening a normally closed circuit for allowing the determination of said inserted device; and said pair of pins are electrodes.

As will be appreciated by those persons skilled in the art, a major advantage provided by the present invention is to have a disposable hygienic shield in a laser handpiece in which proper installation is detected. Additional objects of the present invention will become apparent from the following description.

The method and apparatus of the present invention will be better understood by reference to the following detailed discussion of specific embodiments and the attached figures which illustrate and exemplify such embodiments.

### DESCRIPTION OF THE DRAWINGS

A specific embodiment of the present invention will be described with reference to the following drawings, wherein:
Figure 1 is a diagrammatic view of an embodiment of the insert of the present invention and an embodiment of the laser handpiece into which it is inserted.
Figure 2 is an orthogonal view of an embodiment of the insert of the present invention.
Figure 3 is another orthogonal view of an embodiment of the insert of the present invention showing filters and O-rings in an exploded view.
Figure 4 is a bottom view of an embodiment of the insert of the present invention.
Figure 5 is a side view of an embodiment of the insert of the present invention taken along axis A-A of Figure 3.
Figure 6 is a side view of an embodiment of the insert of the present invention taken along axis B-B of Figure 3.
Figure 7 is an electrical schematic of an embodiment of a portion of the handpiece of the present invention.

### DESCRIPTION OF THE PREFERRED EMBODIMENT

The following preferred embodiment as exemplified by the drawings is illustrative of the invention and is not intended to limit the invention as encompassed by the claims of this application. A device adapted for insertion into a laser handpiece is disclosed herein.

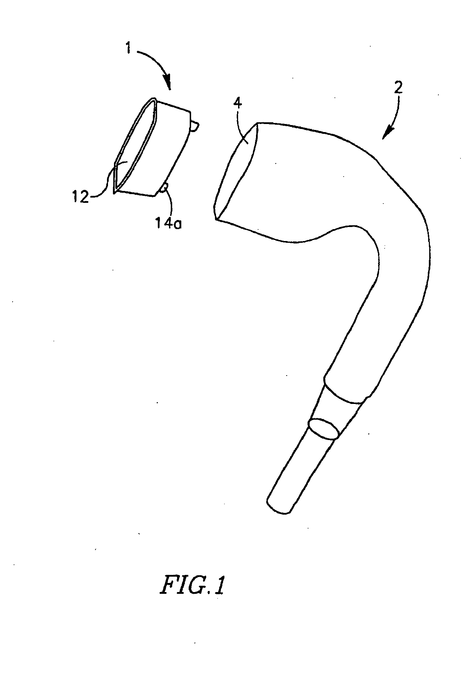

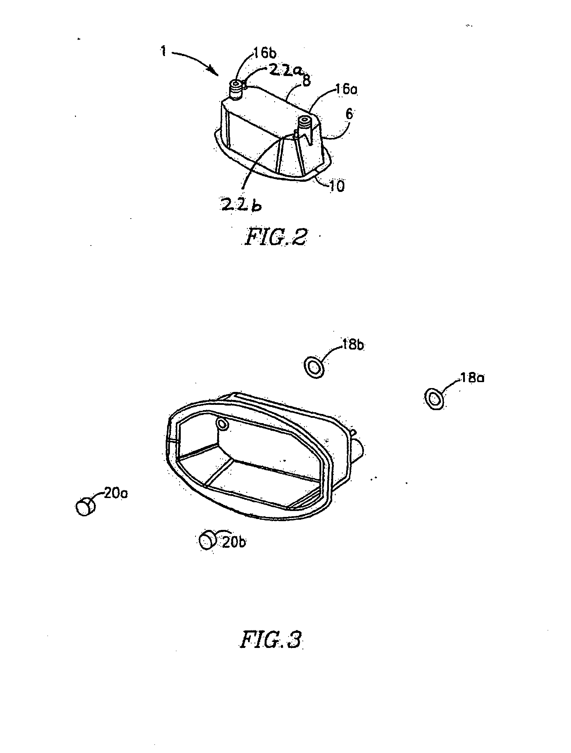

The insert 1, as illustrated generally in Figures 1- 6 is for fitting into a laser handpiece 2. The insert acts, at least in part, as a hygiene shield for one time use on a patient. The laser handpiece has a cavity 4 into which the insert is configured for fitting. The cavity is adapted for applying a vacuum when the handpiece is applied to the skin for treatment. Laser light of particular wavelength and energy is directed to the skin while the skin is under vacuum with the handpiece in a predetermined controlled manner for selected skin treatment.

The insert 1 has an outer wall 6 which conforms to the cavity 4 of the handpiece. Typically, the thickness of the wall is in the range of from about 0.875 mm to about 1.125 mm. The insert has a base 8 which acts as a window through which the laser beam is directed. Typically, the thickness of the base is in the range of from about 0.875 mm to about 1.125 mm. Accordingly, at least the base is manufactured of a material optically transparent to the particular laser wavelength being applied. Preferably, the entire insert is made of the same material (for example, a polycarbonate having 88% light transmission and 1% haze at 2.5 mm) because laser light is reflected and redirected throughout the handpiece cavity by the walls of the cavity and thus also through the outer wall 6 of the insert. Preferably, the insert may be formed of clear GE Lexan HF 1140 Polycarbonate, FDA tripartite (ISO i0993-1 mod)/USP Class VI compliant, UL94V-2; or clear GE Lexan HP1, FDA food contact compliant, UL94V-2.

The base may also include optical properties either applied to or as part of its structure. Such optical properties may include, for example, a diffraction pattern, a refractive property, or an optical coating. Of course, as is well known to those skilled in the art, the base may be made of a different thickness or optical properties than the walls of the insert.

A further alternative to the body is to include either permanent or removable reflective surfaces along at least a portion of the side walls of the insert. The reflective surface, which may extend throughout the interior or exterior surfaces of the insert cavity and which may be either a coating or a removable insertion may be made of, or a substrate coated with, gold and/or other reflective, metals and /or non-metals. The reflective surfaces may be specular, diffuse, or somewhere in between.

The insert due to its exposure to laser energy necessitates the use of a material with at least a Flame Class Rating of UL 94V-2 or (3) CSA. Since the insert comes in contact with the patient's skin, a biocompatible or FDA food contact compliant material is also needed. In addition to conforming plastics, glass is also a suitable material.

The outer wall extends from the base 8 to a peripheral flange 10 which extends around the periphery of the insert. The flange 10 is preferably conformable to the skin of the patient. It is preferable that the flange not extend beyond the handpiece body, except for a portion that is exposed to the side relief of the handpiece body for easing finger access for removal of the insert. Having the flange conform to the outer profile of the handpiece body allows the insert to remain in place when the handpiece is removed from a pocket into which it is preferably stored and calibrated. The outer wall thus forms a cavity 12 within the insert itself. The flange 10 is that portion of the insert that contacts the skin of the patient and forms a sufficient seal so that a vacuum, for example, ranging from about 11 in. Hg (37 kPa.) to about 28 in. Hg (95 kPa.), can be formed therewith. The vacuum thus formed preferably draws the skin into the cavity 12 of the insert before each pulse of electromagnetic radiation is applied and releases the skin upon completion of the pulse.

A channel 14a is formed into the insert for allowing vacuum communication between the handpiece and the cavity 12 of the insert. Preferably, such a channel is formed using a tubular member 16a extending from the base. It is further preferable that another similarly shaped member 16b be symmetrically formed on the base thereby forming another symmetrically placed channel 14b. It is still further preferable that only one of the two channels be operative at any one time. For example, although the insert mates exactly with the cavity 4 of the handpiece, and the two tubular members 16a and 16b fit into mating slots within the handpiece, only one of the members (for example, 16a) may be connected to a vacuum pump. Therefore, only one channel (for example, 14a) would allow passage for a vacuum to be applied to the cavity 12 of the insert when a seal is formed by the flange with the skin. The other tubular member (for example, 16b) would preferably fit into an inoperative channel of the handpiece (not shown). (Of course, as is well known in the art, both channels may alternatively be used for drawing a vacuum either simultaneously or in a sequence.)

In order to make a tight, leak-free sealing engagement with the vacuum channel of the handpiece, it may be preferable to include o-rings 18a and 18b on the tubular members 16a and 16b, respectively. The o-rings are preferably made of elastomeric material as is well known to those skilled in the art. It is further preferable that a 70-durometer silicone elastomer be used. Alternately, other durometers and other elastomers, including Buna-N, Viton, and other rubber equivalents may also be used.

The compression of the o-rings between the sealing surfaces of the handpiece and insert provides sufficient frictional resistance to secure the insert within the handpiece cavity during use. Other latching means such as mating protrusions and depressions integral to the insert and handpiece (or components within the handpiece) could also be used for this purpose. The body may also include a latching mechanism for securing the insert into the handpiece.

It may be preferable to include particle filters 20a and 20b inserted within the channels 14a and 14b, respectively. The preferred filter material is hydrophobic polyethylene, 3 mm thick, 45-90 micron pore size. Other suitable filter materials include polypropylene, Nylon, Teflon, glass, sintered metals and non-flammable grades of cellulose, preferably Porex X-4904 High Density Polyethylene meeting FDA 21 CFR 177.1520. Materials in different thicknesses, such as a woven or matted fabric or a wire mesh, with other pore sizes may also be acceptable for use in this application. Alternatively features such as a series of closely spaced protrusions or an array of small holes in a membrane-like element spanning the vacuum channel could be integrated into the design of the insert itself to replace the particle filters described in the current invention. The addition of the filters into their respective channels may help to protect the vacuum system from clogging with skin debris and other detritus that may be produced during the skin treatment procedure.

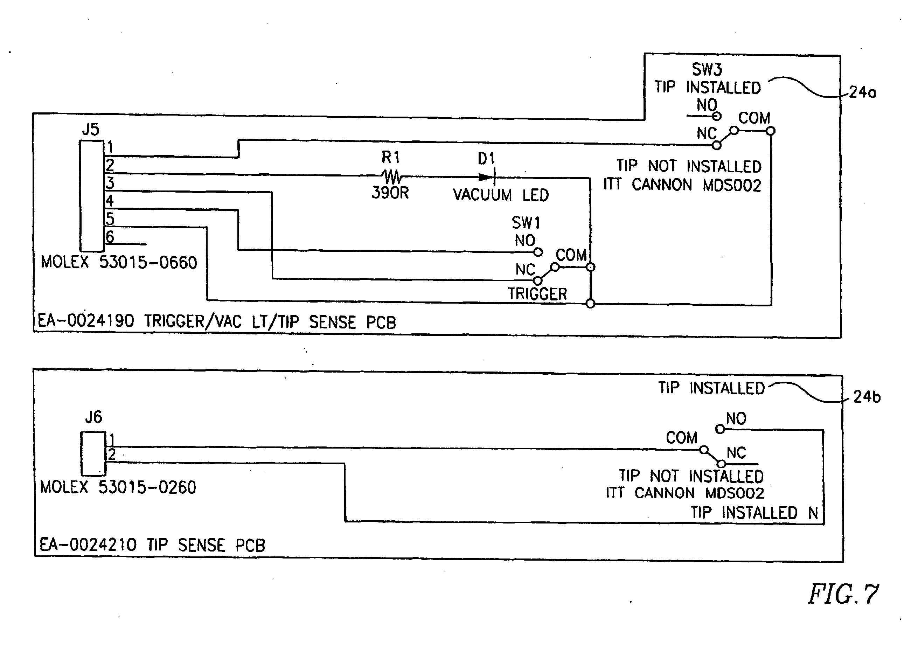

Tip pins 22a and 22b may be symmetrically placed extending from the base of the insert. These pins may be formed to matingly insert into pin receiving holes formed in the cavity of the handpiece. Within the pin receiving holes of the handpiece, which are formed to receive and engage with the tip pins 22a and 22b, are pin detectors for detecting the presence of both of the pins. The detection of the presence of both of the tip pins allows an additional level of safety for the operator and for the patient Of course, as is well known in the art, alternatively, only one of the pin receiving holes may have a pin detector. A further alternative may exclude pins entirely and merely detect the presence of the tubular extensions 16a and 16b when they are inserted into their corresponding channels in the handpiece. Such detection of the tubular channels for conducting a vacuum would be in accordance with the electrical schematic diagram discussed with respect to Figure 7.

Figure 7 shows an electrical schematic diagram of an embodiment of pin detection. By providing this type of pin detection, a safety interlock with the laser is effected. As illustrated, one pin, for example 22a, may fit into a pin-receiving hole of the handpiece in which a normally closed detection switch is located The other pin, for example, 22b, may correspondingly fit into another pin receiving hole of the handpiece in which a normally open detection switch is located. When the insert is properly secured into the handpiece, both of the pins must engage with their respective detection switches in order to allow the laser to become operative. In this particular embodiment described and illustrated in Figure 7, when pin 22a matingly engages with its receiving hole, a pin detection switch 24a is moved from its normally closed position to an open position. Correspondingly, when pin 22b matingly engages with its receiving hole, a pin detection switch 24b is moved from its normally open position to a closed position. When the circuit in which switch 24a is broken and when the circuit in which 24b is completed are both detected, the safety interlock allows the laser to fire. Only when both circuits are detected, with switch 24a being open and switch 24b being closed, is the laser allowed to fire. This arrangement is preferable because lit ensures that misdetection will not result in allowing the laser to fire.

Alternatively, the detection switches may be differently arranged. For example, switches 24a and 24b may both be normally closed switches. As a further alternative, switches 24a and 24b may both be normally open switches.

A further alternative to having pins extending from the insert into receiving holes of the insert may be to have a pin or pins in the handpiece extend into receiving holes or receiving depressions in the insert. In such an embodiment, the safety interlock would determine whether or not the pins extend from the handpiece in the correct manner. In such an embodiment, only when the pins correctly extend from the handpiece into their respective corresponding receptors in the insert, would the laser be allowed to fire.

A still further embodiment of the present invention may include using a conductive material to complete a circuit between two electrodes in the handpiece cavity. All or a selected portion of the insert may be conductive. Thus, when the insert in properly placed in the handpiece, a circuit is completed for establishing proper fit Alternatively, the pins 22a and 22b may be conducting elements or electrodes.

A yet further embodiment of the present invention may include a capacitive or optical proximity detector for non-contact sensing of the insert. An additional embodiment may further include a means for detecting an optical signature of the base. Such detection of the optical signature of the base may include, for example, detecting the reflection signature or the absorption signature of the base for an indication of correct insertion of the tip into the handpiece.

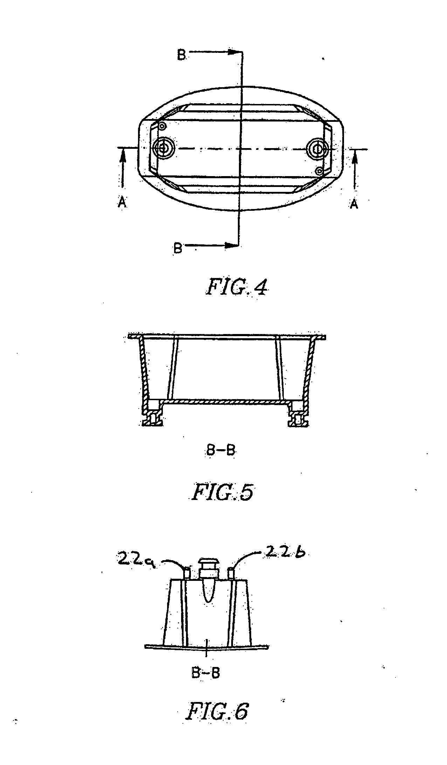

It is preferable that the insert be symmetrical about axes A-A and B-B of Figure 4. In this way, the insert can initially be placed in the cavity of the handpiece easily. Further, since an embodiment of the present invention may have only one of the two channels (for example, 14a) operative for drawing a vacuum, if one filter, for example 20a becomes clogged, the same insert can be easily and quickly repositioned in the handpiece thereby using the other channel (for example, 14b) with the unused filter, for example, 20b. After the insert has been used on a patient, it is disposed of so that the next patient may have a new hygienic insert installed for his use. Reuse of a cleaned sterilized insert is also within the scope of this invention.

Although the particular embodiments shown and described above will prove to be useful in many applications in the skin treatment arts and laser applications in general, to which the present invention pertains, further modifications of the present invention will occur to persons skilled in the art. All such modifications are deemed to be within the scope of the present invention as defined by the appended claims.

## Claims

1. A device configured for insertion into a handpiece (2) having a laser, said device comprising a body (1), said body (1) comprising:
a base (8);
an outer wall (6) extending from a first side of the base (8) and along a periphery of the base (8) to define a cavity (12) within the body (1);
a peripheral flange (10) around a periphery of the outer wall (6); and
at one least channel (14a,b);
said base (8) is of a material transparent to at least one optical wavelength of the laser;
said at least one channel (14a,b) is configured to provide vacuum communication between said cavity (12) and said handpiece (2);
wherein the peripheral flange (10) is configured to form a seal around a section of skin tissue when applied to the section of skin tissue; and
wherein the body (1) is configured to draw the section of skin tissue into the cavity (12) when a vacuum is pulled through the at least one channel (14a,b) by said handpiece (2).
**characterized in that**,
the device further comprises a safety interlock for allowing the laser to fire only when the device is inserted correctly into the handpiece, said safety interlock comprising:;
at least one pair of pins (22a,b);
wherein said at least one pair of pins (22a,b), when said device is inserted into said handpiece (2), provide said safety lock, through one or more of:
(i) at least one of said pair of pins (22a,b) is for completing a normally open circuit for allowing the determination of said inserted device;
(ii) at least one of said pair of pins (22a,b) is for opening a normally closed circuit for allowing the determination of said inserted device;
(iii) one of said pair of pins (22a,b) is for completing a normally open circuit and the other of said pair of pins opens a normally closed circuit for allowing the determination of the said inserted device;
(iv) said one pair of pins (22a,b) are for completing a normally open circuit for allowing the determination of the inserted device;
(iv) said pair of pins (22a,b) are for opening a normally closed circuit for allowing the determination of said inserted device; and
(v) said pair of pins (22a,b) are electrodes.

2. The device of claim 1, said at least one channel (14a,b) including a pair of channels (14a,b) for providing vacuum communication between said cavity (12) and said handpiece (2), wherein each of said pair of channels (14a,b) is adapted for including a particle filter (20a,b), and wherein the device further comprises at least one means for allowing the determination of said inserted device into said handpiece, said determining means (22a,b) including a pair of pins that, when the device is inserted into said handpiece, said pair of pins are determined to be in said handpiece.

3. The device of claim 1, wherein said base (8) includes an optical element and
wherein the optical element includes one or more of:
(i) a lens;
(ii) a diffraction grating or pattern;
(iii) a refractive element;
(iv) an optical coating wherein the body (1) includes a reflecting surface; and
(v) wherein the body (1) includes a latching mechanism.

4. The device of claim 1, wherein the handpiece is configured to pull a vacuum in the cavity (12) to draw the section of skin tissue into the cavity (12) prior to each application of the laser

5. The device of claim 1 wherein each of said at least one channels (14a,b) is adapted for receiving a particle filter (20a,b).

6. The device of claim 1, the at least one channel (14a,b) including at least one member (16a,b) extending from a second side of the base (8).

7. The device of claim 1, wherein the channels (14a,b) are symmetric about an axis of the device.

8. The device of claim 1, wherein the outer wall (6) includes a reflecting surface.

9. The device of claim 1, wherein the body (1) includes a latching mechanism.

10. The device of claim 1, wherein said determination means comprises at least one pair of pins (22a, 22b) and one pair of receiving depressions in said insert, such that said pins (22a, 22b) extend from said handpiece into their respective corresponding receptors in said insert.

## Patentansprüche

1. Vorrichtung, die zum Einsetzen in ein Handstück (2) mit einem Laser konfiguriert ist, wobei die Vorrichtung einen Körper (1) umfasst, wobei der Körper (1) Folgendes umfasst:
einen Boden (8);
eine Außenwand (6), die sich von einer ersten Seite des Bodens (8) und längs eines Umfangs des Bodens (8) erstreckt, um einen Hohlraum (12) innerhalb des Körpers (1) abzugrenzen;
einen Umfangsflansch (10) um einen Umfang der Außenwand (6) herum und
mindestens einen Kanal (14a, 14b);
wobei der Boden (8) aus einem Material ist, das für mindestens eine optische Wellenlänge des Lasers durchlässig ist;
wobei der mindestens eine Kanal (14a, 14b) dazu konfiguriert ist, eine Vakuumverbindung zwischen dem Hohlraum (12) und dem Handstück (2) bereitzustellen;
wobei der Umfangsflansch (10) dazu konfiguriert ist, eine Dichtung um einen Hautgewebeabschnitt herum zu bilden, wenn er auf den Hautgewebeabschnitt aufgebracht wird; und
wobei der Körper (1) dazu konfiguriert ist, den Hautgewebeabschnitt in den Hohlraum (12) zu ziehen, wenn ein Vakuum durch den mindestens einen Kanal (14a, 14b) durch das Handstück (2) erzeugt wird,
**dadurch gekennzeichnet, dass**
die Vorrichtung weiterhin eine Sicherheitssperre umfasst, um dem Laser das Feuern nur zu ermöglichen, wenn die Vorrichtung korrekt in das Handstück eingesetzt ist, wobei die Sicherheitssperre Folgendes umfasst:
mindestens ein Paar Stifte (22a, 22b);
wobei das mindestens eine Paar Stifte (22a, 22b), wenn die Vorrichtung in das Handstück (2) eingesetzt ist, die Sicherheitssperre durch eines oder mehrere der folgenden bereitstellt:
(i) mindestens einer des Paars Stifte (22a, 22b) ist zum Schließen eines normalerweise offenen Schaltkreises zum Ermöglichen der Bestimmung der eingesetzten Vorrichtung;
(ii) mindestens einer des Paars Stifte (22a, 22b) ist zum Öffnen eines normalerweise geschlossenen Schaltkreises zum Ermöglichen der Bestimmung der eingesetzten Vorrichtung;
(iii) einer des Paars Stifte (22a, 22b) ist zum Schließen eines normalerweise offenen Schaltkreises und der andere des Paars Stifte ist zum Öffnen eines normalerweise geschlossenen Schaltkreises zum Ermöglichen der Bestimmung der eingesetzten Vorrichtung;
(iv) das eine Paar Stifte (22a, 22b) ist zum Schließen eines normalerweise offenen Schaltkreises zum Ermöglichen der Bestimmung der eingesetzten Vorrichtung;
(v) das Paar Stifte (22a, 22b) ist zum Öffnen eines normalerweise geschlossenen Schaltkreises zum Ermöglichen der Bestimmung der eingesetzten Vorrichtung und
(vi) bei dem Paar Stifte (22a, 22b) handelt es sich um Elektroden.

2. Vorrichtung nach Anspruch 1, wobei der mindestens eine Kanal (14a, 14b) ein Paar Kanäle (14a, 14b) zum Bereitstellen einer Vakuumverbindung zwischen dem Hohlraum (12) und dem Handstück (2) beinhaltet, wobei jeder des Paars Kanäle (14a, 14b) dazu eingerichtet ist, einen Partikelfilter (20a, 20b) zu beinhalten, und wobei die Vorrichtung weiterhin mindestens ein Mittel zum Ermöglichen der Bestimmung der eingesetzten Vorrichtung in das Handstück umfasst, wobei das Bestimmungsmittel (22a, 22b) ein Paar Stifte beinhaltet, so dass, wenn die Vorrichtung in das Handstück eingesetzt ist, das Paar Stifte als in dem Handstück bestimmt wird.

3. Vorrichtung nach Anspruch 1, wobei der Boden (8) ein optisches Element beinhaltet und wobei das optische Element eines oder mehrere der folgenden beinhaltet:
(i) eine Linse;
(ii) ein Beugungsgitter oder -muster;
(iii) ein Brechungselement;
(iv) eine optische Beschichtung, wobei der Körper (1) eine reflektierende Oberfläche beinhaltet; und
(v) wobei der Körper (1) einen Arretiermechanismus beinhaltet.

4. Vorrichtung nach Anspruch 1, wobei das Handstück dazu konfiguriert ist, ein Vakuum in dem Hohlraum (12) zu erzeugen, um den Hautgewebeabschnitt vor jeder Anwendung des Lasers in den Hohlraum (12) zu ziehen.

5. Vorrichtung nach Anspruch 1, wobei jeder des mindestens einen Kanals (14a, 14b) dazu eingerichtet ist, einen Partikelfilter (20a, 20b) aufzunehmen.

6. Vorrichtung nach Anspruch 1, wobei der mindestens eine Kanal (14a, 14b) mindestens ein Element (16a, 16b) beinhaltet, das sich von einer zweiten Seite des Bodens (8) erstreckt.

7. Vorrichtung nach Anspruch 1, wobei die Kanäle (14a, 14b) um eine Achse der Vorrichtung symmetrisch sind.

8. Vorrichtung nach Anspruch 1, wobei die Außenwand (6) eine reflektierende Oberfläche beinhaltet.

9. Vorrichtung nach Anspruch 1, wobei der Körper (1) einen Arretiermechanismus beinhaltet.

10. Vorrichtung nach Anspruch 1, wobei das Bestimmungsmittel mindestens ein Paar Stifte (22a, 22b) und ein Paar Aufnahmevertiefungen in dem Einsatz umfasst, so dass die Stifte (22a, 22b) sich von dem Handstück in ihre jeweiligen entsprechenden Aufnahmeteile in dem Einsatz erstrecken.

## Revendications

1. Dispositif configuré pour être inséré dans une pièce à main (2) comportant un laser, ledit dispositif comprenant un corps (1), ledit corps (1) comportant :
un support (8) ;
une paroi extérieure (6) s'étendant depuis un premier côté du support (8) et le long d'une périphérie du support (8) pour définir une cavité (12) au sein du corps (1) ;
une collerette périphérique (10) autour d'une périphérie de la paroi extérieure (6) ; et un canal au moins (14a, b) ;
ledit support (8) est fait d'une matière transparente sur au moins une longueur d'onde optique du laser ;
ledit un canal au moins (14a, b) est configuré pour prévoir une communication sous vide entre ladite cavité (12) et ladite pièce à main (2) ;
dans lequel la collerette périphérique (10) est configurée de sorte à former un joint étanche autour d'une partie du tissu cutané quand elle est appliquée sur la partie du tissu cutané, et
dans lequel le corps (1) est configuré de façon à insérer la partie de tissu cutané dans la cavité (12) quand un vide est insufflé dans ledit un canal au moins (14a, b) par ladite pièce à main (2),
**caractérisé en ce que**,
le dispositif comprend en outre un verrouillage de sécurité pour permettre au laser d'émettre des rayons, ledit verrouillage de sécurité comportant :
une paire de goupilles au moins (22a, b) ;
dans lequel ladite une paire de goupilles au moins (22a, b), quand ledit dispositif est inséré dans la pièce à main (2), ladite paire de goupilles (22a, b) assure ledit verrouillage de sécurité, par l'un ou plusieurs des postes suivants, à savoir :
(i) au moins une de ladite paire de goupilles (22a, b) a pour objet de boucler un circuit normalement ouvert permettant de déterminer ledit dispositif inséré ;
(ii) au moins une de ladite paire de goupilles (22a, b) a pour objet d'ouvrir un circuit normalement fermé permettant de déterminer ledit dispositif inséré ;
(iii) une de ladite paire de goupilles (22a, b) a pour objet de boucler un circuit normalement ouvert et l'autre paire de goupilles ouvre un circuit normalement fermé permettant de déterminer ledit dispositif inséré ;
(iv) lesdites paires de goupilles (22a, b) ont pour objet de boucler un circuit normalement ouvert permettant de déterminer le dispositif inséré ;
(iv) lesdites paires de goupilles (22a, b) ont pour objet d'ouvrir un circuit normalement fermé permettant de déterminer ledit dispositif inséré ; et
(v) lesdites paires de goupilles (22a, b) sont des électrodes.

2. Dispositif selon la revendication 1, ledit un canal au moins (14a, b) disposant d'une paire de canaux (14a, b) pour prévoir une communication sous vide entre ladite cavité (12) et ladite pièce à main (2) ; dans lequel chacune desdites paires de canaux (14a, b) est conçue pour inclure un filtre à particules (20a, b), et dans lequel le dispositif comprend en outre au moins un moyen permettant de déterminer ledit dispositif inséré dans ladite pièce à main, ledit moyen de détermination (22a, b) comprenant une paire de goupilles, quand le dispositif est inséré dans ladite pièce à main, le dites paires de goupilles sont déterminées être dans ladite pièce à main.

3. Dispositif selon la revendication 1, dans lequel ledit support (8) inclut un élément optique et dans lequel l'élément optique inclut l'un ou plusieurs des postes suivants, à savoir :
(i) une lentille ;
(ii) un réseau ou un spectre de diffraction :
(iii) un élément réfractif ;
(iv) un revêtement optique dans lequel le corps (1) inclut une surface réfléchissante, et
(v) dans lequel le corps (1) inclut un mécanisme de verrouillage.

4. Dispositif selon la revendication 1, dans lequel la pièce à main est configurée pour insuffler un vide dans la cavité (12) pour insérer la partie du tissu cutané dans la cavité (12) avant chaque application du laser

5. Dispositif selon la revendication 1 dans lequel chacun desdits un canal au moins (14a, b) est conçu pour recevoir un filtre à particules (20a, b).

6. Dispositif selon la revendication 1, ledit un canal au moins (14a, b) inclut au moins un membre (16a, b) s'étendant depuis un second côté du support (8).

7. Dispositif selon la revendication 1, dans lequel les canaux (14a, b) sont symétriques par rapport à un axe du dispositif.

8. Dispositif selon la revendication 1, dans lequel la paroi extérieure (6) inclut une surface réfléchissante.

9. Dispositif selon la revendication 1, dans lequel le corps (1) inclut°un mécanisme de verrouillage.

10. Dispositif selon la revendication 1, dans lequel ledit moyen de détermination comprend au moins une paire de goupilles (22a, 22b) et une paire de cavités de logement dans ledit insert, de sorte que lesdites goupilles (22a, 22b) s'étendent de ladite pièce à main jusqu'aux récepteurs correspondants situés dans ledit insert.
